(19) Europäisches Patentamt
European Patent Office
Office européen des brevets

(11) Veröffentlichungsnummer: **0 203 504 A2**

(12) **EUROPÄISCHE PATENTANMELDUNG**

(21) Anmeldenummer: **86106871.6**

(22) Anmeldetag: **21.05.86**

(51) Int. Cl.⁴: **A 01 N 43/653**, A 01 N 43/50, C 07 D 405/06, C 07 D 409/06

(30) Priorität: **31.05.85 DE 3519454**

(43) Veröffentlichungstag der Anmeldung: **03.12.86 Patentblatt 86/49**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Elbe, Hans-Ludwig, Dr., Dasnöckel 59, D-5600 Wuppertal 11 (DE)**
Erfinder: **Jautelat, Manfred, Dr., Müllersbaum 28, D-5093 Burscheid (DE)**
Erfinder: **Büchel, Karl Heinz, Prof.Dr., Dabringhausener Strasse 42, D-5093 Burscheid (DE)**
Erfinder: **Reinecke, Paul, Dr., Steinstrasse 8, D-5090 Leverkusen 3 (DE)**
Erfinder: **Hänssler, Gerd, Dr., Am Arenzberg 58a, D-5090 Leverkusen 3 (DE)**

(54) **Vinylazole zur Bekämpfung von Mikroorganismen.**

(57) Verwendung von teilweise bekannten Vinylazolen der Formel

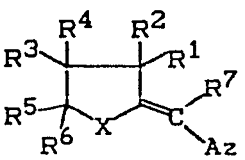

(I)

in welcher

$R^1$, $R^2$, $R^3$, $R^4$, $R^5$ und $R^6$ unabhängig voneinander jeweils für Wasserstoff, Alkyl, Halogenalkyl oder Halogen stehen, wobei bis zu maximal 3 dieser Reste für Halogen und/oder Halogenalkyl stehen können, oder auch

$R^1$ und $R^2$ oder

$R^1$ und $R^3$ jeweils gemeinsam für einen zweifach verknüpften Alkandiylrest stehen,

$R^7$ für Wasserstoff oder Halogen steht,

X für Sauerstoff, Schwefel, eine Sulfinylgruppe oder eine Sulfonylgruppe steht,

Az für Imidazolyl oder Triazolyl steht, sowie deren Säureadditionssalzen und Metallsalz-Komplexen zur Bekämpfung von unerwünschten Mikroorganismen.

EP 0 203 504 A2

BAYER AKTIENGESELLSCHAFT 5090 Leverkusen, Bayerwerk
Konzernverwaltung RP
Patentabteilung Dü/Ke-c
IIa

BEZEICHNUNG GEÄNDERT
Siehe Titelseite

Mittel zur Bekämpfung von Mikroorganismen

Die Erfindung betrifft die Verwendung von teilweise bekannten Vinylazolen zur Bekämpfung von unerwünschten Mikroorganismen.

Es ist bereits bekannt, daß bestimmte organische Schwefel-Verbindungen fungizide Eigenschaften besitzen (vgl. z.B. K.H. Büchel, "Pflanzenschutz und Schädlingsbekämpfung", S. 137ff, G. Thieme Verlag, Stuttgart 1977). So kann zum Beispiel Zink-ethylen-1,2-bis-dithiocarbamat zur Bekämpfung von Fungi eingesetzt werden. Die Wirksamkeit dieses Stoffes ist jedoch insbesondere bei niedrigen Aufwandmengen nicht immer in allen Anwendungsbereichen völlig zufriedenstellend.

Außerdem sind bestimmte Azolyltetrahydrofuran-2-ylidenmethan-Derivate und deren Einsatz als Zwischenprodukte beschrieben worden (vgl. DE-OS 3 336 861). Eine fungizide Wirksamkeit dieser Stoffe ist aber bisher nicht bekannt.

Es wurde nun gefunden, daß die teilweise bekannten Vinylazole der allgemeinen Formel

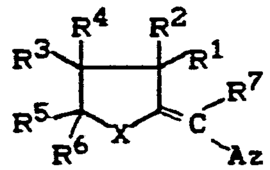

(I)

in welcher

$R^1$, $R^2$, $R^3$, $R^4$, $R^5$ und $R^6$ unabhängig voneinander jeweils für Wasserstoff, Alkyl, Halogenalkyl oder Halogen stehen, wobei bis zu maximal 3 dieser Reste für Halogen und/oder Halogenalkyl stehen können, oder auch

$R^1$ und $R^2$ oder

$R^1$ und $R^3$ jeweils gemeinsam für einen zweifach verknüpften Alkandiylrest stehen,

$R^7$ für Wasserstoff oder Halogen steht,

X für Sauerstoff, Schwefel, eine Sulfinylgruppe oder eine Sulfonylgruppe steht,

Az für Imidazolyl oder Triazolyl steht,

sowie deren Säureadditionssalze und Metallsalz-Komplexe sehr gut zur Bekämpfung von unerwünschten Mikroorganismen geeignet sind.

Die Verbindungen der Formel (I) können als geometrische Isomere oder Isomerengemische unterschiedlicher Zusammensetzung vorliegen. Die Erfindung betrifft sowohl die Verwendung von reinen Isomeren als auch die Verwendung von Isomerengemischen.

Überraschenderweise lassen sich die erfindungsgemäßen Stoffe besser zur Bekämpfung von unerwünschten Mikroorganismen einsetzen als die kontitutionell ähnlichsten Verbindungen, die aus dem Stand der Technik vorbekannt sind. Darüberhinaus zeigen die erfindungsgemäßen Stoffe auch eine stärkere fungizide Wirksamkeit als das Zink-ethylen-1,2-bis-dithiocarbamat, welches eine wirkungsmäßig naheliegende Verbindung ist.

Die erfindungsgemäß zu verwendenden Vinylazole sind durch die Formel (I) allgemein definiert. Bevorzugt sind Verbindungen der Formel (I), in denen

$R^1$, $R^2$, $R^3$, $R^4$, $R^5$ und $R^6$ unabhängig voneinander jeweils für Wasserstoff, Fluor, Chlor, Brom, geradkettiges oder verzweigtes Alkyl mit 1 bis 4 Kohlenstoffatomen oder für geradkettiges oder verzweigtes Halogenalkyl mit 1 bis 2 Kohlenstoffatomen und 1 bis 5 gleichen oder verschiedenen Halogenatomen stehen, wobei jedoch nur bis zu 3 dieser Reste für Fluor, Chlor, Brom und/oder für Halogenalkyl stehen können,

oder auch

$R^1$ und $R^2$ oder

$R^1$ und $R^3$ jeweils gemeinsam für einen geradkettigen oder verzweigten zweifach verknüpften Alkandiylrest mit 2 bis 10 Kohlenstoffatomen stehen,

$R^7$ für Wasserstoff, Chlor oder Brom steht,

X für Sauerstoff, Schwefel, eine Sulfinylgruppe oder eine Sulfonylgruppe steht und

Az für Imidazol-1-yl oder 1,2,4-Triazol-1-yl steht.

Besonders bevorzugt sind Verbindungen der Formel (I), in denen

$R^1$, $R^2$, $R^3$, $R^4$, $R^5$ und $R^6$ unabhängig voneinander jeweils für Wasserstoff, Methyl, Ethyl, Fluor, Chlor, Brom, Chlormethyl, Dichlormethyl, Trichlormethyl, Fluormethyl, Difluormethyl, Trifluormethyl, Dichlorfluormethyl oder Difluorchlormethyl stehen, wobei jedoch nur bis zu 3 dieser Reste für Fluor, Chlor, Brom, Chlormethyl, Dichlormethyl, Trichlormethyl, Fluormethyl, Difluormethyl, Trifluormethyl, Dichlorfluormethyl und/oder Difluorchlormethyl stehen, oder auch

$R^1$ und $R^2$ gemeinsam für Butan-1,4-diyl oder Pentan-1,5-diyl stehen, oder

$R^1$ und $R^3$ gemeinsam für Propan-1,3-diyl oder Butan-1,4-diyl stehen,

$R^7$ für Wasserstoff, Chlor oder Brom steht,

X für Sauerstoff, Schwefel, eine Sulfinylgruppe oder eine Sulfonylgruppe steht und

Az für Imidazol-1-yl oder 1,2,4-Triazol-1-yl steht.

Im einzelnen seien außer den bei den Herstellungsbeispielen genannten Verbindungen die folgenden Verbindungen der allgemeinen Formel (I) genannt:

Tabelle 1

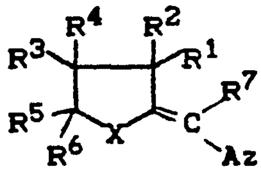

(I)

(Az ist jeweils 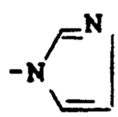 oder 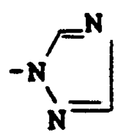 )

| $R^1$ | $R^2$ | $R^3$ | $R^4$ | $R^5$ | $R^6$ | $R^7$ | X |
|---|---|---|---|---|---|---|---|
| $CH_3$ | $CH_3$ | H | H | H | H | Cl | O |
| $C_2H_5$ | $C_2H_5$ | H | H | H | H | H | O |
| $C_2H_5$ | $C_2H_5$ | H | H | H | H | H | SO |
| $C_2H_5$ | H | H | H | H | H | H | S |

Tabelle 1 (Fortsetzung)

| $R^1$ | $R^2$ | $R^3$ | $R^4$ | $R^5$ | $R^6$ | $R^7$ | X |
|---|---|---|---|---|---|---|---|
| $CH_3$ | $CH_3$ | H | H | H | H | Br | $SO_2$ |
| $CH_3$ | $CH_3$ | H | H | H | H | Cl | $SO_2$ |
| $C_2H_5$ | $C_2H_5$ | H | H | H | H | H | $SO_2$ |
| $F-CH_2-$ | $CH_3$ | H | H | H | H | H | O |
| $F-CH_2-$ | $F-CH_2-$ | H | H | H | H | H | O |
| $F-CH_2-$ | $F-CH_2-$ | H | H | H | H | H | $SO_2$ |
| $F-CH_2-$ | $CH_3$ | H | H | H | H | H | $SO_2$ |
| $ClCH_2-$ | $CH_3$ | H | H | H | H | H | O |
| $ClCH_2-$ | $CH_3$ | H | H | H | H | H | $SO_2$ |
| $F-CH_2-$ | $CH_3$ | H | H | H | H | Cl | O |
| $F-CH_2-$ | $CH_3$ | H | H | H | H | Br | O |
| $F-CH_2-$ | $F-CH_2-$ | H | H | H | H | Cl | O |
| $F-CH_2-$ | $F-CH_2-$ | H | H | H | H | Br | O |
| $F-CH_2-$ | $CH_3$ | H | H | H | H | Cl | $SO_2$ |
| $F-CH_2-$ | $CH_3$ | H | H | H | H | Br | $SO_2$ |
| $F-CH_2-$ | $F-CH_2-$ | H | H | H | H | Cl | $SO_2$ |
| $F-CH_2-$ | $F-CH_2-$ | H | H | H | H | Br | $SO_2$ |
| $F-CH_2-$ | $F-CH_2-$ | H | H | H | H | H | SO |
| $F-CH_2-$ | $CH_3$ | H | H | H | H | H | SO |
| $F-CH_2-$ | $F-CH_2-$ | H | H | H | H | H | S |
| $F-CH_2-$ | $CH_3$ | H | H | H | H | H | S |
| $CH_3$ | $CH_3$ | H | Br | H | H | Cl | O |
| $CH_3$ | $CH_3$ | H | Br | H | H | Br | O |
| $CH_3$ | $CH_3$ | H | Cl | H | H | Cl | O |
| $CH_3$ | $CH_3$ | H | Cl | H | H | Br | O |
| $CH_3$ | $CH_3$ | H | H | H | $CH_2Br-$ | H | $SO_2$ |
| $CH_3$ | $CH_3$ | H | H | H | $CH_2Cl-$ | H | O |

Tabelle 1 (Fortsetzung)

| $R^1$ | $R^2$ | $R^3$ | $R^4$ | $R^5$ | $R^6$ | $R^7$ | X |
|---|---|---|---|---|---|---|---|
| $CH_3$ | $CH_3$ | H | H | H | $CH_2Br$- | Cl | O |
| $-(CH_2)_5-$ | | H | H | H | H | H | O |
| $-(CH_2)_5-$ | | H | H | H | H | H | SO |
| $-(CH_2)_5-$ | | H | H | H | H | H | O |
| $-(CH_2)_5-$ | | H | H | H | H | H | $SO_2$ |

Tabelle 2

| $R^1$ | $R^3$ | $R^2$ | $R^4$ | $R^5$ | $R^6$ | $R^7$ | X |
|---|---|---|---|---|---|---|---|
| $-(CH_2)_4-$ | | $CH_3$ | H | H | H | H | O |
| $-(CH_2)_4-$ | | $CH_3$ | H | H | H | H | $SO_2$ |
| $-(CH_2)_4-$ | | $CH_3$ | H | H | H | Cl | O |
| $-(CH_2)_4-$ | | $CH_3$ | H | H | H | Br | O |
| $-(CH_2)_4-$ | | $CH_3$ | H | H | H | Cl | $SO_2$ |
| $-(CH_2)_4-$ | | $CH_3$ | H | H | H | Br | $SO_2$ |
| $-(CH_2)_4-$ | | $CH_3$ | H | H | H | H | S |

Bevorzugte erfindungsgemäß verwendbare Verbindungen sind auch Additionsprodukte aus Säuren und denjenigen Vinylazolen der Formel (I), in denen die Substituenten $R^1$, $R^2$, $R^3$, $R^4$, $R^5$, $R^6$, $R^7$, X und Az die Bedeutungen haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und die Bromwasserstoffsäure, insbesondere die Chlorwasserstoffsäure, ferner Phosphorsäure, Salpetersäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Vinylazolen der Formel (I), in denen die Substituenten $R^1$, $R^2$, $R^3$, $R^4$, $R^5$, $R^6$, $R^7$, X und Az die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu

pflanzenverträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Die erfindungsgemäß verwendbaren Vinylazole der Formel (I) sind teilweise bekannt (vgl. DE-OS 3 204 795, DE-OS 3 229 734, DE-OS 3 336 861, DE-OS 3 336 859). Sie können nach den dort beschriebenen Verfahren hergestellt werden.

Noch nicht bekannt sind Vinylazole der Formel

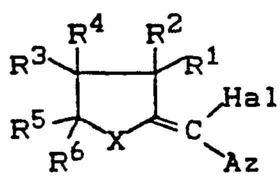

(Ia)

in welcher

$R^1$, $R^2$, $R^3$, $R^4$, $R^5$, $R^6$, X und Az die oben angegebene Bedeutung haben und

Hal für Halogen steht.

Man erhält Verbindungen der Formel (Ia), wenn man Vinylazole der Formel

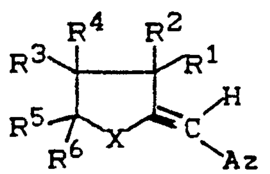

(Ib)

in welcher

$R^1$, $R^2$, $R^3$, $R^4$, $R^5$, $R^6$, X und Az die oben angegebene Bedeutung haben

mit Halogenen, gegebenenfalls in Gegenwart eines Verdünnungsmittels, umsetzt und die so erhältlichen halogenierten Azolverbindungen der Formel

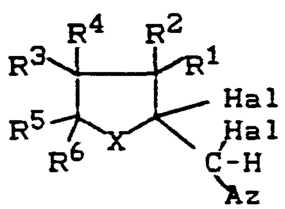

(II)

in welcher

$R^1$, $R^2$, $R^3$, $R^4$, $R^5$, $R^6$, X und Az die oben angegebene Bedeutung haben und

Hal für Halogen steht,

entweder nach vorhergehender Isolierung in einer 2. Stufe, oder direkt anschließend an die 1. Stufe in einem sogenannten "Eintopfverfahren" mit starken Basen gegebenenfalls in Gegenwart eines Verdünnungsmittels dehydrohalogeniert.

Als Verdünnungsmittel für diese Umsetzung kommen anorganische oder organische Lösungsmittel in Frage. Vorzugsweise verwendbar sind Wasser, Alkohole, wie Methanol oder Ethanol, oder deren Gemische mit Wasser, oder chlorierte Kohlenwasserstoffe, wie Dichlormethan, Chloroform oder Tetrachlorkohlenstoff.

Als Basen kommen bei der obigen Umsetzung vorzugsweise Alkalimetallhydroxide, wie Natrium- oder Kaliumhydroxid, oder Alkalimetallalkoholate, wie Natriummethylat, Natriumethylat oder Kalium-tert.-butylat, in Betracht.

Die Reaktionstemperaturen können bei der obigen Umsetzung innerhalb eines größeren Bereiches variiert werden. Im allgemeinen arbeitet man bei Temperaturen zwischen 0°C und 100°C, vorzugsweise zwischen 20°C und 50°C.

Die obige Umsetzung wird im allgemeinen unter Normaldruck durchgeführt. Es ist jedoch auch möglich, unter erhöhtem Druck zu arbeiten.

Zur Durchführung der obigen Umsetzung setzt man auf 1 Mol an Vinylazol der Formel (Ib) im allgemeinen 1,0 bis 1,5 Mol, vorzugsweise äquimolare Mengen an Halogen und Base ein. Die Durchführung der Umsetzung, die Aufarbeitung des Reaktionsgemisches und die Isolierung der Verbindungen der Formel (Ia) erfolgt nach üblichen Methoden.

Die als Ausgangsstoffe für die oben beschriebene Herstellung der Vinylazole der Formel (Ia) erforderlichen Vinylazole der Formel (Ib) sind teilweise bekannt (vgl. z.B. DE-OS 3 336 861).

Noch nicht bekannt sind Vinylazole der Formel

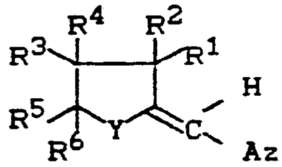

(Ic)

in welcher

$R^1$, $R^2$, $R^3$, $R^4$, $R^5$, $R^6$ und Az die oben angegebene Bedeutung haben und

Y für Schwefel, eine Sulfinylgruppe oder eine Sulfonylgruppe steht.

Verbindungen der Formel (Ic) lassen sich herstellen, indem man

a) Vinylchlorid-Derivate der Formel

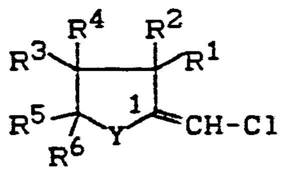

(III)

in welcher

$R^1$, $R^2$, $R^3$, $R^4$, $R^5$ und $R^6$ die oben angegebene Bedeutung haben und

$Y^1$ für eine Sulfinyl- oder Sulfonylgruppe steht,

mit Azolen der Formel

$$Az\text{-}H \qquad (IV)$$

in welcher

Az die oben angegebene Bedeutung hat,

gegebenenfalls in Gegenwart eines Verdünnungsmittels und gegebenenfalls in Gegenwart eines Säurebindemittels umsetzt,

oder

b) die nach Verfahren (a) erhältlichen Sulfinyl-Vinylazole der Formel

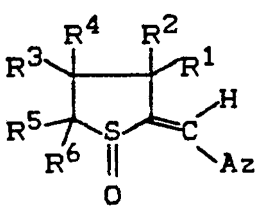

(Id)

in welcher

$R^1$, $R^2$, $R^3$, $R^4$, $R^5$, $R^6$ und Az die oben angegebene Bedeutung haben,

entweder

α) nach üblichen Verfahren, gegebenenfalls in Gegenwart eines Verdünnungsmittels, am Schwefel der Sulfinylgruppe oxidiert, oder

β) ebenfalls nach üblichen Verfahren, gegebenenfalls in Gegenwart eines Verdünnungsmittels, am Schwefel der Sulfinylgruppe reduziert.

Die zur Durchführung des Herstellungsverfahrens (a) als Ausgangsstoffe benötigten Vinylchlorid-Derivate der Formel (III) sind ebenfalls noch nicht bekannt. Man erhält sie, wenn man 1-Penten-Derivate der Formel

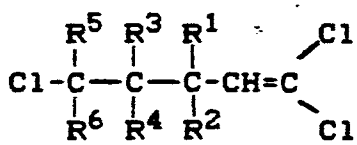

(V)

in welcher

$R^1$, $R^2$, $R^3$, $R^4$, $R^5$ und $R^6$ die oben angegebene Bedeutung haben,

zunächst in einer 1.Stufe mit Thioessigsäure der Formel

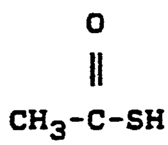

(VI)

gegebenenfalls in Gegenwart eines Verdünnungsmittels, wie beispielsweise N,N-Dimethylformamid, und gegebenenfalls in Gegenwart einer Base, wie beispielsweise Natriummethylat, bei Temperaturen zwischen +50°C und +200°C umsetzt, und die so erhältlichen Tetrahydrothiophenderivate der Formel

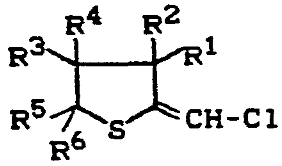

(VII)

in welcher

$R^1$, $R^2$, $R^3$, $R^4$, $R^5$ und $R^6$ die oben angegebene Bedeutung haben,

nach üblichen Methoden, beispielsweise mit Wasserstoffperoxid, gegebenenfalls in Gegenwart eines Verdünnungsmittels, wie beispielsweise Eisessig, bei Temperaturen zwischen -20°C und +70°C entweder partiell (bis zur Sulfoxid-Stufe; d.h. mit stöchiometrischen Mengen an Oxidationsmittel, Temperatur -20°C bis +30°C) oder vollständig (bis zur Sulfonstufe; d.h. mit einem Überschuß an Oxidationsmittel, Temperatur 20°C bis 70°C, gegebenenfalls unter Verwendung eines Katalysators, wie beispielsweise Ammoniummolybdat) oxidiert.

Die 1-Penten-Derivate der Formel (V) sind bekannt (vgl. z.B. DE-OS 3 336 861). Die Thioessigsäure der Formel (VI) und die zur Durchführung des Herstellungsverfahrens (a) weiterhin als Ausgangsstoffe benötigten Azole der Formel (IV) sind allgemein bekannte Verbindungen der organischen Chemie.

Als Verdünnungsmittel zur Durchführung des Herstellungsverfahrens (a) kommen inerte organische Lösungsmittel in Frage.

Vorzugsweise verwendbar sind aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton, Butanon oder Methylisopropylketon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, oder Alkohole wie Methanol, Ethanol oder Propanol.

Das Herstellungsverfahren (a) wird gegebenenfalls in Gegenwart eines Säurebindemittels durchgeführt. Als solche kommen alle üblichen anorganischen oder organischen Basen in Frage. Vorzugsweise verwendbar sind Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen(DBU).

Man kann auch einen entsprechenden Überschuß an dem gleichzeitig als Reaktionspartner eingesetzten Azol der Formel (IV) als Säurebindemittel verwenden.

Die Reaktionstemperaturen können bei der Durchführung des Herstellungsverfahrens (a) in einem größeren Bereich variiert werden. Im allgemeinen arbeitet man bei Temperaturen zwischen 20°C und 150°C, vorzugsweise bei Temperaturen zwischen 60°C und 120°C.

Zur Durchführung des Herstellungsverfahrens (a) setzt man pro Mol an Vinylchlorid-Derivat der Formel (III) im allgemeinen 1,0 bis 10,0 Mol, vorzugsweise 1,0 bis 5,0 Mol an Azol der Formel (IV) und gegebenenfalls 1,0 bis 5,0 Mol an Säurebindemittel ein. Die Reaktionsdurchführung, Aufarbeitung und Isolierung der Reaktionsprodukte der Formel (Ic) erfolgt nach üblichen Verfahren.

Als Oxidationsmittel zur Durchführung des Herstellungsverfahrens (b-α) kommen alle üblicherweise für Schwefeloxidationen verwendbaren Oxidationsmittel in Frage. Vorzugsweise verwendbar sind Peroxide, wie Wasserstoffperoxid,Peressigsäure, Perpropionsäure oder gegebenenfalls substituierte Perbenzoesäuren, wie m-Chlorperbenzoesäure oder p-Nitroperbenzoesäure.

Das Herstellungsverfahren (b-α) wird gegebenenfalls in Gegenwart eines geeigneten Verdünnungsmittels durchgeführt. Vorzugsweise in Betracht kommen Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Carbonsäuren oder Anhydride, wie Essigsäure, Propionsäure oder Acetanhydrid, oder dipolar aprotische Lösungsmittel, wie Acetonitril, Aceton, Essigsäureethylester oder Dimethylformamid.

Die Reaktionstemperaturen können bei dem Herstellungsverfahren (b-α) in gewissen Bereichen variiert werden. Im allgemeinen arbeitet man bei Temperaturen zwischen -20°C und +70°C vorzugsweise zwischen 0°C und +50°C.

Zur Durchführung des Herstellungsverfahrens (b-α) setzt man pro Mol an Sulfinyl-vinylazol der Formel (Id) im allgemeinen 0,8 bis 1,5 Mol, vorzugsweise 1,0 bis 1,2 Mol an Oxidationsmittel ein. Die Reaktionsführung, Aufarbeitung und Isolierung der oxidierten Endprodukte der Formel (Ic) erfolgt nach üblichen Verfahren.

Als Reduktionsmittel zur Durchführung des Herstellungsverfahrens (b-β) kommen alle üblicherweise für Sulfoxidreduktionen verwendbaren anorganischen und organischen Reduktionsmittel in Frage. Beispielhaft genannt seien Iodwasserstoffsäure und 2,4-Bis(4-methoxy-phenyl)-1,3-dithia-2,4-diphosphetan-2,4-dithion (Lawesson's Reagenz), (vgl. auch Houben-Weyl "Methoden der organischen Chemie", Band IX, S.218ff, 4.Auflage, Thieme Verlag, Stuttgart 1955).

Als Verdünnungsmittel zur Durchführung des Herstellungsverfahrens (b-β) kommen inerte organische oder anorganische Lösungsmittel in Frage.

Vorzugsweise in Betracht kommen aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Amide, wie Dimethylformamid, Dimethylacetamid, N-Methylpyrrolidon oder Hexamethylphosphorsäuretriamid, Alkohole, wie Methanol,

Ethanol oder Propanol oder deren Gemische mit Wasser. Gegebenenfalls kommt auch Wasser als Verdünnungsmittel in Betracht.

Die Reaktionstemperaturen können bei der Durchführung des Herstellungsverfahrens (b-ß) in einem größeren Bereich variiert werden. Im allgemeinen arbeitet man bei Temperaturen zwischen 0°C und +150°C, vorzugsweise zwischen +20°C und +120°C.

Zur Durchführung des Herstellungsverfahrens (b-ß) setzt man pro Mol an Sulfinyl-vinylazol der Formel (Id) im allgemeinen 1,0 bis 15,0 Mol, vorzugsweise 1,0 bis 10,0 Mol an Reduktionsmittel ein. Die Reaktionsdurchführung, Aufarbeitung und Isolierung der Endprodukte der Formel (Ic) erfolgt nach üblichen Verfahren (vgl. in diesem Zusammenhang beispielsweise Org. Prep. Proced. Int. Band 16, S.171-198 [1984]; Synthesis 1984, 827-829; Houben-Weyl "Methoden der organischen Chemie", Band IX, S. 218ff, 4. Auflage, Thieme Verlag Stuttgart 1955),

Zur Herstellung von Säureadditionssalzen der Verbindungen der Formel (I) kommen vorzugsweise folgende Säuren in Frage: Halogenwasserstoffsäuren, wie z.B. Chlorwasserstoffsäure und Bromwasserstoffsäure, insbesondere Chlorwasserstoffsäure, ferner Phosphorsäure, Salpetersäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe des Periodensystems der Elemente in Frage, wobei Kupfer, Zink, Mangan, Magnesium, Zinn, Eisen und Nickel beispielhaft genannt seien.

Als Anionen von Salzen kommen solche in Betracht, die sich vorzugsweise von folgenden Säuren ableiten: Halogenwasserstoffsäuren, wie z.B. Chlorwasserstoffsäure und Bromwasserstoffsäure, ferner Phosphorsäure, Salpetersäure und Schwefelsäure.

Die Metallsalzkomplexe von Verbindungen der Formel (I) können in einfacher Weise nach üblichen Verfahren erhalten werden, so z.B. durch Lösen des Metallsalzes in Alkohol, z.B. Ethanol, und Hinzufügen zur Verbindung der Formel (I). Man kann Metallsalzkomplexe in bekannter Weise, z.B. durch Abfiltrieren, isolieren und gegebenenfalls durch Umkristallisation reinigen.

Die erfindungsgemäß verwendbaren Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet, insbesondere als Fungizide und Bakterizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Pythium-Arten, wie beispielsweise Pythium ultimum:
Phytophthora-Arten, wie beispielsweise Phytophthora infestans;
Pseudoperonospora-Arten, wie beispielsweise Pseudoperonospora humuli oder Pseudoperonospora cubense:
Plasmopara-Arten, wie beispielsweise Plasmopara viticola:
Peronospora-Arten, wie beispielsweise Peronospora pisi oder P. brassicae;

Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea;
(Konidienform: Drechslera, Syn: Helminthosporium);
Cochliobolus-Arten, wie beispielsweise Cochliobolus sativus;
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielseise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäß verwendbaren Wirkstoffe mit besonders gutem Erfolg zur Bekämpfung von Getreidekrankheiten, wie beispielsweise gegen den Erreger des Getreidemehltaus (Erysiphe graminis) oder gegen den Erreger der Streifenkrankheit der Gerste (Drechslera graminea), oder gegen den Erreger der Braunfleckenkrankheit (Cochliobolus sativus) oder gegen den Erreger der Netzfleckenkrankheit der Gerste (Pyrenophora teres), gegen Rost-Arten, Septoria-Arten, gegen Fusarium culmorum, zur Bekämpfung von Reiskrankheiten, wie beispielsweise gegen den Erreger Pyricularia oryzae oder Pellicularia sasakii oder zur Bekämpfung von Obst- und Gemüsekrankheiten wie beispielsweise gegen echten Mehltau, Schorf oder Botrytis anwenden.
Neben der protektiven Wirkung zeichnen sich die erfindungsgemäß verwendbaren Wirkstoffe insbesondere auch durch eine gute systemische Wirksamkeit aus.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungiziden, Insektiziden, Bakteriziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder den daraus durch weiteres Verdünnen bereiteten Anwendungsformen, wie gebrauchsfertige Lösungen, Emulsionen, Suspensionen, Pulver, Pasten und Granulate, angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Anwendung als Fungizide können die Wirkstoffkonzentrationen bei der Behandlung von Pflanzenteilen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 1,0 Gew.-%, vorzugsweise von 0,0001 bis 0,02 Gew.-%, am Wirkungsort erforderlich.

Die Herstellung und die Verwendung der erfindungsgemäßen Stoffe wird durch die folgenden Beispiele veranschaulicht.

## Herstellungsbeispiele

### Beispiel 1

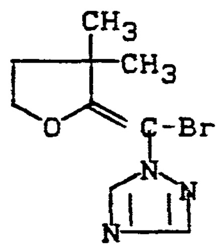

17,9 g (0,1 Mol) (3,3-Dimethyl-tetrahydrofuran-2-yl-iden)-(1,2,4-triazol-1-yl)-methan in 150 ml Chloroform werden bei Raumtemperatur tropfenweise unter Rühren mit 16 g (0,1 Mol) Brom versetzt. Nach beendeter Zugabe wird eine Stunde nachgerührt, und das Reaktionsgemisch wird unter vermindertem Druck eingeengt. Der verbleibende Rückstand wird in Wasser aufgenommen. Es wird mit 2 normaler wäßriger Natronlauge auf pH 7 eingestellt und mehrfach mit Ether extrahiert. Die vereinigten Etherphasen werden mit Wasser gewaschen, über Natriumsulfat getrocknet und unter vermindertem Druck vom Lösungsmittel befreit.

Man erhält 4,7 g (14 % der Theorie) an (3,3-Dimethyl-tetrahydrofuran-2-yliden)-(1,2,4-triazol-1-yl)-brommethan vom Schmelzpunkt 122°C.

Beispiel 2

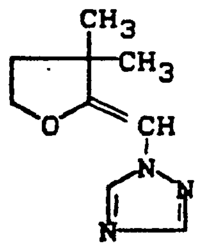

85 g (1,23 Mol) 1,2,4-Triazol und 464 g (33,6 Mol) Kaliumcarbonat werden in 2,5 l Aceton vorgelegt. Bei Raumtemperatur läßt man ohne Kühlung unter Rühren 220 g (1,12 Mol) 1,5-Dichlor-3,3-dimethyl-2-pentanon zutropfen. Nach Beendigung der Zugabe läßt man 2 Stunden bei Rückflußtemperatur nachrühren.

Das Reaktionsgemisch wird abgekühlt, über eine Nutsche abgesaugt, und die Mutterlauge wird unter vermindertem Druck eingeengt. Der Rückstand wird in Methylenchlorid aufgenommen. Die Lösung wird mit Wasser gewaschen, über Natriumsulfat getrocknet und eingeengt. Der verbleibende Rückstand wird in Petrolether verrührt, abgesaugt und im Vakuum bei 50°C getrocknet.

Man erhält 145,3 g (56,5 % der Theorie) (3,3-Dimethyl-tetrahydrofuran-2-yliden)-(1,2,4-triazol-1-yl)-methan vom Schmelzpunkt 47°C.

Herstellung der Ausgangsverbindungen

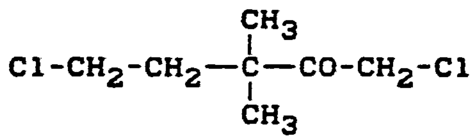

In 476 g (3,25 Mol) 2-Chlormethylen-3,3-dimethyltetrahydrofuran wird unter Eiskühlung ein starker Chlorwasserstoffgasstrom aus einer Bombe eingeleitet. Das Gas wird vollständig absorbiert, und die Temperatur des Reaktionsgemisches steigt bis auf 30°C. Nach vollständiger Sättigung mit Chlorwasserstoff wird das Reaktionsgemisch 2 Stunden bei Raumtemperatur nachgerührt. Überschüssiger Chlorwasserstoff wird zunächst in die Wasserstrahlpumpe gezogen; dann wird bei gutem Vakuum destilliert.

Man erhält 531 g (90 % der Theorie) 1,5-Dichlor-3,3-dimethyl-2-pentanon vom Siedepunkt 85-90°C/0,3 mbar.

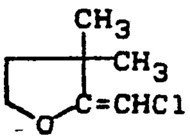

806 g (4 Mol) 1,1,5-Trichlor-3,3-dimethyl-1-penten werden mit 360 ml (4,4 Mol) wasserfreiem Natriumacetat in 1 l Dimethylformamid 6 Stunden unter Rückfluß erhitzt. Nach Abkühlen auf 100°C tropft man 1,6 l (8 Mol) 30 %ige Natriummethylatlösung in Methanol ein und erhitzt weitere 4 Stunden unter Rückfluß. Die kalte Lösung wird in Wasser gegossen und mit Methylenchlorid mehrfach extrahiert. Nach Trocknen der organischen Phase und Abdestillieren des Lösungsmittels bleiben 654 g Produkt zurück, das über eine Kolonne fraktioniert wird.

Man erhält 522 g (89 % der Theorie) 2-Chlormethylen-3,3-dimethyl-tetrahydrofuran vom Siedepunkt 84-87°C/20 mbar.

Beispiel 3

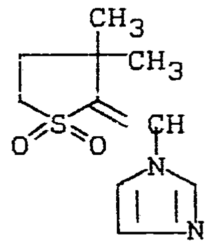

73,6 g (0,378 Mol) 3,3-Dimethyl-2-chlormethylentetrahydrothiophen-1,1-dioxid, 38,6 g (0,57 Mol) Imidazol und 78,4 g (0,57 Mol) Kaliumcarbonat in 200 ml Dimethylformamid werden 15 Stunden bei 140° C gerührt. Zur Aufarbeitung gießt man die erkaltete Reaktionsmischung in Wasser, extrahiert mehrfach mit Methylenchlorid, wäscht die vereinigten organischen Phasen mit Wasser, trocknet über Natriumsulfat und entfernt das Lösungsmittel unter vermindertem Druck.

Man erhält 33,8 g (40 % der Theorie) an (3,3-Dimethyl-1,1-dioxo-tetrahydrothiophen-2-yliden)-(imidazol-1-yl)-methan vom Schmelzpunkt 78° C.

Herstellung der Ausgangsverbindungen

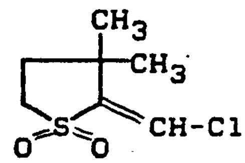

Zu 85 g (0,52 Mol) 3,3-Dimethyl-2-chlormethylen-tetrahydrothiophen in 1000 ml Eisessig gibt man unter Rühren bei Raumtemperatur 296 g (2,8 Mol) 30-prozentige wässrige Wasserstoffperoxidlösung. Nach beendeter Zugabe rührt man 20 Stunden bei 50°C, gießt die erkaltete Reaktionsmischung in 3000 ml Wasser, extrahiert mehrfach mit Dichlormethan, wäscht die vereinigten Dichlormethanphasen mit Wasser, trocknet über Natriumsulfat und entfernt das Lösungsmittel unter vermindertem Druck. Der Rückstand wird aus Diisopropylether umkristallisiert.

Man erhält 78,4 g (74,2 % der Theorie) an 3,3-Dimethyl-2-chlormethylen-tetrahydrothiophen-1,1-dioxid vom Schmelzpunkt 62°C.

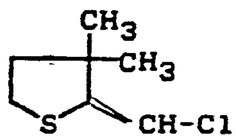

806 g (4,0 Mol) 3,3-Dimethyl-1,1,5-trichlor-1-penten, 344 g (4,4 Mol) Thioessigsäure und 880 ml (4,4 Mol) 30-prozentige methanolische Natriummethylatlösung werden in 4000 ml Dimethylformamid 3,5 Stunden unter Rückfluß erhitzt. Dann gibt man weitere 440 ml (2,2 Mol) 30-prozentige methanolische Natriummethylatlösung zu und erhitzt weitere 7 Stunden unter Rückfluß. Zur Aufarbeitung destilliert man die Hauptmenge des Lösungsmittels unter vermindertem Druck ab, nimmt den Rückstand in Wasser auf, extrahiert mehrfach mit Dichlormethan, wäscht die vereinigten Dichlormethanphasen mit Wasser, trocknet über Natriumsulfat und entfernt das Lösungsmittel unter vermindertem Druck. Der Rückstand wird im Hochvakuum fraktioniert.

Man erhält 466 g (72 % der Theorie) an 3,3-Dimethyl-2-chlormethylen-tetrahydrothiophen vom Siedepunkt 38°-45°C bei 0,1 mbar als E/Z-Isomerengemisch.

Z-Isomeres: NMR($CDCl_3$): δ 1,2 (s, 6 H), 1,95 (t, J=6,5 Hz, 2 H), 3,05 (t, J=6,5 Hz, 2 H), 5,85 (s, 1 H) ppm

E-Isomeres: NMR($CDCl_3$): δ 1,35 (s, 6 H), 2,05 (t, J=6,5 Hz, 2 H), 3,0 (t, J=6,5 Hz, 2 H), 5,75 (s, 1 H) ppm

Beispiel 4

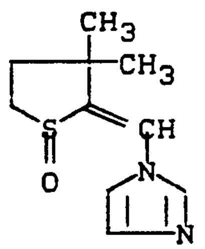

21,5 g (0,12 Mol) 3,3-Dimethyl-2-chlormethylen-tetrahydrothiophen-1-oxid, 12,3 g (0,18 Mol) Imidazol und 24,9 g (0,18 Mol) Kaliumcarbonat werden in 130 ml Dimethylformamid 12 Stunden bei 0°C gerührt. Zur Aufarbeitung gibt man 300 ml Wasser zu, extrahiert mehrfach mit Dichlormethan, wäscht die vereinigten Dichlormethanphasen mit Wasser, trocknet über Natriumsulfat und entfernt das Lösungsmittel unter vermindertem Druck. Der Rückstand wird über eine Kieselgelsäule (Laufmittel: Essigester/Cyclohexan 3:1) chromatographisch gereinigt.

Man erhält 11 g (46 % der Theorie) an (3,3-Dimethyl-1-oxo-tetrahydrothiophen-2-yliden)-(imidazol-1-yl)-methan vom Brechungsindex $n_D^{20}$ 1,580.

Herstellung der Ausgangsverbindung

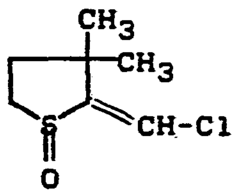

Zu 110 g (0,678 Mol) 3,3-Dimethyl-2-chlormethylen-tetrahydrothiophen in 110 ml Eisessig gibt man tropfenweise unter Rühren bei 0°C bis 5°C 55 ml (62,6 g, 0,65 Mol) 35-prozentige wässrige Wasserstoffperoxidlösung, rührt nach beendeter Zugabe eine weitere Stunde bei 0° bis 5°C und anschließend 4 Stunden bei Raumtemperatur. Zur Aufarbeitung gießt man die Reaktionsmischung in Wasser, neutralisiert mit wäßriger Natronlauge und extrahiert mehrfach mit Dichlormethan. Die vereinigten Dichlormethanphasen werden mit Wasser gewaschen, über Natriumsulfat getrocknet und unter vermindertem Druck vom Lösungsmittel befreit. Der Rückstand wird aus 500 ml Diisopropylether umkristallisiert.

Man erhält 72,6 g (40 % der Theorie) an 3,3-Dimethyl-2-chlormethylen-tetrahydrothiophen-1-oxid vom Schmelzpunkt 78-79°C.

Beispiel 5

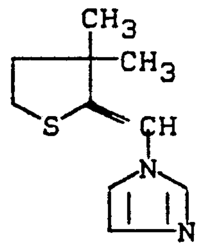

2,1 g (0,01 Mol) (3,3-Dimethyl-1-oxo-tetrahydrothiophen-2-yliden)-(imidazol-1-yl)-methan werden 4 Stunden lang bei 70°C in 10 ml konzentrierter Iodwasserstoffsäure gerührt. Zur Aufarbeitung gibt man zu der erkalteten Reaktionsmischung 100 ml Wasser, stellt mit verdünnter wäßriger Natronlauge auf pH 6 ein, extrahiert mehrfach mit Dichlormethan, wäscht die vereinigten Dichlormethanphasen mit Wasser, trocknet über Natriumsulfat, entfernt das Lösungsmittel unter vermindertem Druck und reinigt den Rückstand chromatographisch (Kieselgel; Laufmittel: Essigester/Cyclohexan = 3:1).

Man erhält 1,5 g (77 % der Theorie) an (3,3-Dimethyl-tetrahydrothiophen-2-yliden)-(imidazol-1-yl)-methan vom Brechungsindex $n_D^{20}$ = 1,646.

Nach den in den vorhergehenden Beispielen beschriebenen Methoden und entsprechend den allgemeinen Angaben zur Herstellung erhält man die in der Tabelle 3 aufgeführten Vinylazole der Formel (I):

Tabelle 3

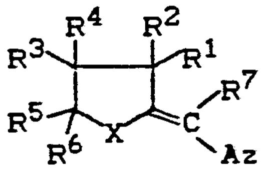

(I)

Tabelle 3

| Beispiel Nr. | $R^1$ | $R^2$ | $R^3$ | $R^4$ | $R^5$ | $R^6$ | $R^7$ | X | Az | physikalische Eigenschaften |
|---|---|---|---|---|---|---|---|---|---|---|
| 6 | $CH_3$ | $CH_3$ | H | H | H | H | H | O | 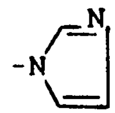 | $n_D^{20}$ 1,5038 |
| 7 | $CH_3$ | $C_2H_5$ | H | H | H | H | H | O | 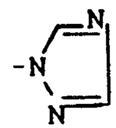 | $n_D^{20}$ 1,5237 |
| 8 | $CH_3$ | $C_2H_5$ | H | H | H | H | H | O | 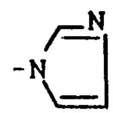 | $n_D^{20}$ 1,5364 |
| 9 | $CH_3$ | $CH_3$ | H | H | H | H | H | O | 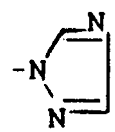 | $n_D^{20}$ 1,5018 *) |
| 10 | $CH_3$ | $CH_3$ | H | H | H | H | H | $SO_2$ | 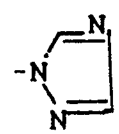 | Fp 128°C |
| 11 | $CH_3$ | $CH_3$ | H | H | $-CH_2Br$ | H | H | O | 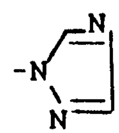 | $n_D^{20}$ 1,5452 |

*) von Beispiel (2)abweichendes E/Z-Isomeren-Verhältnis

Tabelle 3 (Fortsetzung)

| Beispiel Nr. | $R^1$ | $R^2$ | $R^3$ | $R^4$ | $R^5$ | $R^6$ | $R^7$ | X | Az | physikalische Eigenschaften |
|---|---|---|---|---|---|---|---|---|---|---|
| 12 | $CH_3$ | $CH_3$ | H | H | H | H | Cl | O | 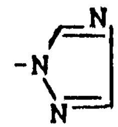 | Fp 100°C |
| 13 | $CH_3$ | $CH_3$ | H | H | H | H | H | SO | 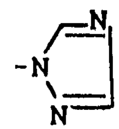 | $n_D^{20}$ 1,547 |
| 14 | $CH_3$ | $CH_3$ | H | H | H | H | H | S | 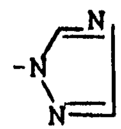 | $n_D^{20}$ 1,635 |

Anwendungsbeispiele:

In den folgenden Anwendungsbeispielen wurde die nachstehend aufgeführte Verbindung als Vergleichssubstanz eingesetzt:

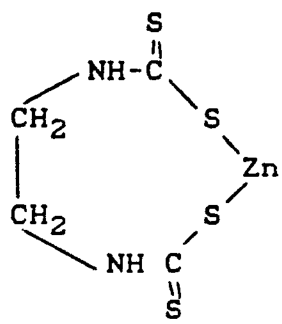 = (A)

Zink-ethylen-1,2-bis-(dithiocarbamat)

**Beispiel A**

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Wirkstoffe, Wirkstoffkonzentrationen und Versuchsergebnisse gehen aus der folgenden Tabelle hervor.

## Tabelle A

Erysiphe-Test Gerste /protektiv

| Wirkstoff | Wirkstoffkonzentration in der Spritzbrühe in Gew.-% | Krankheitsbefall in % der unbehandelten Kontrolle |
|---|---|---|
| 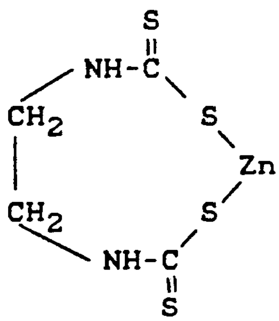 (A)<br>(bekannt) | 0,025 | 100 |
| 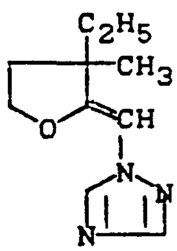 (7) | 0,025 | 41,2 |

Tabelle A (Fortsetzung)

Erysiphe-Test Gerste /protektiv

| Wirkstoff | Wirkstoffkonzentration in der Spritzbrühe in Gew.-% | Krankheitsbefall in % der unbehandelten Kontrolle |
|---|---|---|
| 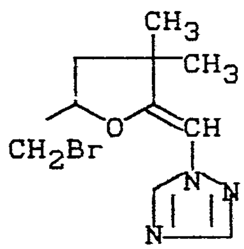 (11) | 0,025 | 33,7 |
| 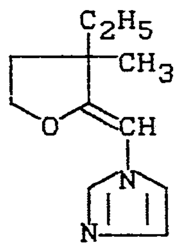 (8) | 0,025 | 0,0 |
| 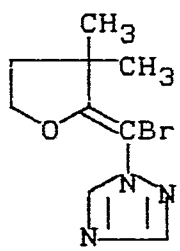 (1) | 0,025 | 33,8 |
| 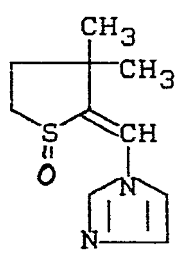 (4) | 0,025 | 12,5 |

## Beispiel B

Drechslera graminea-Test (Gerste) / Saatgutbehandlung
(syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4°C aus. Dabei wird die Keimung der Gerste und gegebenenfalls auch der Pilzsporen eingeleitet. Anschließend sät man die vorgekeimte Gerste mit 2 x 50 Korn 3 cm tief in eine Standarderde und kultiviert sie im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Wirkstoffe, Wirkstoffmengen und Versuchsergebnisse gehen aus der folgenden Tabelle hervor.

Tabelle B

Drechslera graminea-Test (Gerste) /Saatgutbehandlung
(syn. Helminthosporium gramineum)

| Wirkstoff | Wirkstoffaufwandmenge in mg/kg Saatgut | kranke Pflanzen in % der insgesamt aufgelaufenen Pflanzen |
|---|---|---|
| ungebeizt | - | 23,4 |
| 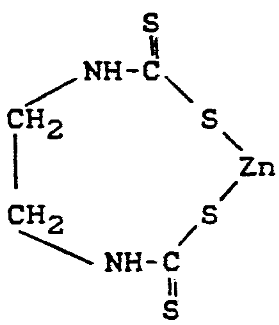 (A) (bekannt) | 500 | 18,0 |
| 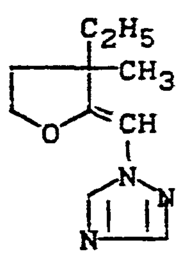 (7) | 500 | 0,0 |

Tabelle B (Fortsetzung)

Drechslera graminea-Test (Gerste) /Saatgutbehandlung
(syn. Helminthosporium gramineum)

| Wirkstoff | Wirkstoffaufwandmenge in mg/kg Saatgut | kranke Pflanzen in % der insgesamt aufgelaufenen Pflanzen |
|---|---|---|
| 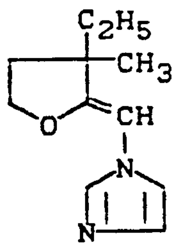 (8) | 500 | 0,0 |

## Beispiel C

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Wirkstoffe, Wirkstoffkonzentrationen und Versuchsergebnisse gehen aus der folgenden Tabelle hervor.

## Tabelle C

Pyricularia-Test (Reis) /protektiv

| Wirkstoff | Wirkstoffkonzentration in der Spritzbrühe in % | Krankheitsbefall in % der unbehandelten Kontrolle |
|---|---|---|
| 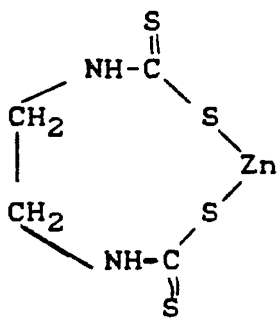 (A)<br>(bekannt) | 0,025 | 75 |
| 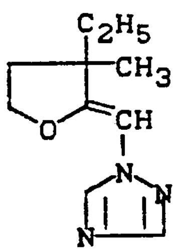 (7) | 0,025 | 30 |

Tabelle C (Fortsetzung)

Pyricularia-Test (Reis) /protektiv

| Wirkstoff | Wirkstoffkonzentration in der Spritzbrühe in % | Krankheitsbefall in % der unbehandelten Kontrolle |
|---|---|---|
| 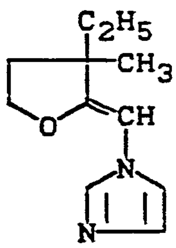 (8) | 0,025 | 0 |

Beispiel D

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Wirkstoffe, Wirkstoffmengen und Versuchsergebnisse gehen aus der folgenden Tabelle hervor.

Tabelle D

Pyricularia-Test (Reis) /systemisch

| Wirkstoff | Aufwandmenge in mg Wirkstoff pro 100 $cm^2$ | Krankheitsbefall in % der unbehandelten Kontrolle |
|---|---|---|
| 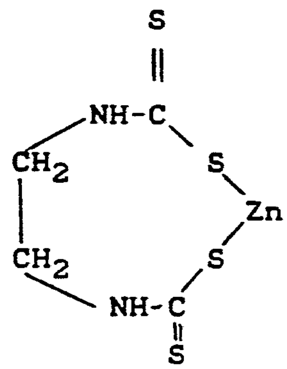 (A) (bekannt) | 100 | 100 |
| 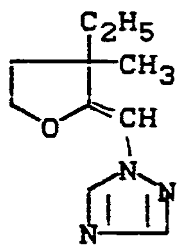 (7) | 100 | 10 |

Tabelle D (Fortsetzung)

Pyricularia-Test (Reis) /systemisch

| Wirkstoff | Aufwandmenge in mg Wirkstoff pro 100 $cm^2$ | Krankheitsbefall in % der unbehandelten Kontrolle |
|---|---|---|
| 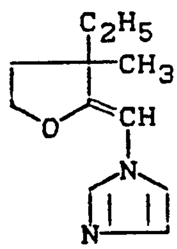 (8) | 100 | 0 |

## Patentansprüche

1. Mittel zur Bekämpfung von Mikroorganismen, gekennzeichnet durch einen Gehalt an mindestens einem Vinylazol der Formel

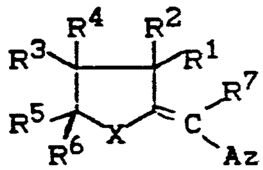

(I)

in welcher

$R^1$, $R^2$, $R^3$, $R^4$, $R^5$ und $R^6$ unabhängig voneinander jeweils

für Wasserstoff, Alkyl, Halogenalkyl oder Halogen stehen, wobei bis zu maximal 3 dieser Reste für Halogen und/oder Halogenalkyl stehen können, oder auch

$R^1$ und $R^2$ oder

$R^1$ und $R^3$ jeweils gemeinsam für einen zweifach verknüpften Alkandiylrest stehen,

$R^7$ für Wasserstoff oder Halogen steht,

X für Sauerstoff, Schwefel, eine Sulfinylgruppe oder eine Sulfonylgruppe steht,

Az für Imidazolyl oder Triazolyl steht,

bzw. eines Säureadditions-Salzes oder Metallsalz-Komplexes eines Vinylazols der Formel (I).

2. Mittel gemäß Anspruch 1, gekennzeichnet durch einen Gehalt an mindestens einem Vinylazol der Formel (I), in welcher

$R^1$, $R^2$, $R^3$, $R^4$, $R^5$ und $R^6$ unabhängig voneinander jeweils für Wasserstoff, Fluor, Chlor, Brom, geradkettiges oder verzweigtes Alkyl mit 1 bis 4 Kohlenstoffatomen oder für geradkettiges oder verzweigtes Halogenalkyl mit 1 bis 2 Kohlenstoffatomen und 1 bis 5 gleichen oder verschiedenen Halogenatomen stehen, wobei jedoch nur bis zu 3 dieser Reste für Fluor, Chlor, Brom und/oder für Halogenalkyl stehen können,

oder auch

$R^1$ und $R^2$ oder

$R^1$ und $R^3$ jeweils gemeinsam für einen geradkettigen oder verzweigten zweifach verknüpften Alkandiylrest mit 2 bis 10 Kohlenstoffatomen stehen,

$R^7$ für Wasserstoff, Chlor oder Brom steht,

X für Sauerstoff, Schwefel, eine Sulfinylgruppe oder eine Sulfonylgruppe steht und

Az für Imidazol-1-yl oder 1,2,4-Triazol-1-yl steht,

bzw. eines Säureadditions-Salzes oder Metallsalz-Komplexes eines solchen Vinylazols.

3. Verfahren zur Bekämpfung von Mikroorganismen, dadurch gekennzeichnet, daß man Vinylazole der Formel (I) bzw. Säureadditions-Salze oder Metallsalz-Komplexe von Vinylazolen der Formel (I) auf Mikroorganismen und/oder deren Lebensraum ausbringt.

4. Verwendung von Vinylazolen der Formel (I) bzw. von Säureadditions-Salzen oder Metallsalz-Komplexen von Vinylazolen der Formel (I) zur Bekämpfung von Mikroorganismen.

5. Verfahren zur Herstellung von Mitteln zur Bekämpfung von Mikroorganismen, dadurch gekennzeichnet, daß man Vinylazole der Formel (I) bzw. deren Säureadditions-Salze oder Metallsalz-Komplexe mit Streckmitteln und/oder oberflächenaktiven Stoffen vermischt.

6. Verwendung gemäß Anspruch 4, dadurch gekennzeichnet, daß man als Vinylazol die Verbindung der Formel

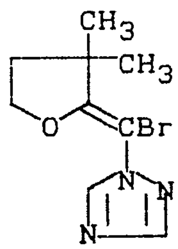

einsetzt.

7. Verwendung gemäß Anspruch 4, dadurch gekennzeichnet, daß man als Vinylazol die Verbindung der Formel

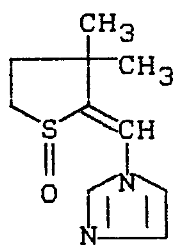

einsetzt.

8. Verwendung gemäß Anspruch 4, dadurch gekennzeichnet, daß man als Vinylazol die Verbindung der Formel

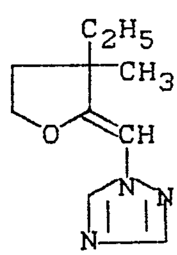

einsetzt.

9. Verwendung gemäß Anspruch 4, dadurch gekennzeichnet, daß man als Vinylazol die Verbindung der Formel

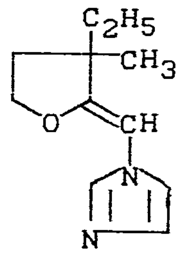

einsetzt.

10. Verwendung gemäß Anspruch 4, dadurch gekennzeichnet, daß man als Vinylazol die Verbindung der Formel

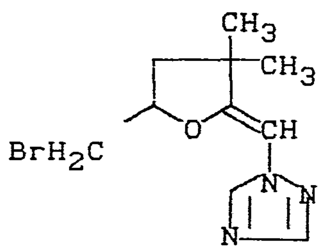

einsetzt.